# EUROPEAN PATENT SPECIFICATION

(11) **EP 3 785 546 B1**
(45) Date of publication and mention of the grant of the patent: **28.09.2022**
(21) Application number: 19214792.4
(22) Date of filing: 10.12.2019
(51) Int. Cl.: A23L 7/104, A21D 8/04

(54) **A NOVEL MULTI-STRAIN COMPOSITE STARTER FOR FLOUR PRODUCTS AND USE THEREOF**
NEUARTIGER MEHRSTAMM-VERBUNDSTARTER FÜR MEHLPRODUKTE UND DESSEN VERWENDUNG
NOUVEAU DÉMARREUR COMPOSITE MULTI-SOUCHE POUR PRODUITS DE FARINE ET SON UTILISATION

(30) Priority: 29.08.2019 CN 201910806793
(43) Date of publication of application: 03.03.2021
(73) Proprietor: Institute of Food Science and Technology, Chinese Academy of Agricultural Sciences, 100193 Peking (CN)
(72) Inventor: Mu, Taihua, Beijing 100193 (CN); Sun, Hongnan, Beijing 100193 (CN); Zhao, Zheng, Beijing 100193 (CN)
(74) Representative: Kutzenberger Wolff & Partner

(56) References cited:
- CA-A1- 3 076 540
- CN-A- 108 354 170
- US-A1- 2019 090 519
- ZHENG ZHAO ET AL: "Microbial characterization of five Chinese traditional sourdoughs by high-throughput sequencing and their impact on the quality of potato steamed bread", FOOD CHEMISTRY, vol. 274, 1 February 2019 (2019-02-01), pages 710-717, XP055708973, NL ISSN: 0308-8146, DOI: 10.1016/j.foodchem.2018.08.143
- ZHENG ZHAO ET AL: "Comparative study of the nutritional quality of potato steamed bread fermented by different sourdoughs", JOURNAL OF FOOD PROCESSING AND PRESERVATION, vol. 43, no. 9, 28 June 2019 (2019-06-28), XP055708978, TRUMBULL, CT, US ISSN: 0145-8892, DOI: 10.1111/jfpp.14080
- HEIDE-MARIE DANIEL ET AL: "in the sourdough microbial ecosystem", ANTONIE VAN LEEUWENHOEK, KLUWER ACADEMIC PUBLISHERS, DO, vol. 99, no. 1, 21 October 2010 (2010-10-21), pages 63-73, XP019876139, ISSN: 1572-9699, DOI: 10.1007/S10482-010-9517-2
- DE VUYST L ET AL: "Microbial ecology of sourdough fermentations: Diverse or uniform?", FOOD MICROBIOLOGY, ACADEMIC PRESS LTD, LONDON, GB, vol. 37, 18 June 2013 (2013-06-18), pages 11-29, XP028770933, ISSN: 0740-0020, DOI: 10.1016/J.FM.2013.06.002
- LI ZHIJIAN ET AL: "Microbiological characterization of traditional dough fermentation starter (Jiaozi) for steamed bread making by culture-dependent and culture-independent methods", INTERNATIONAL JOURNAL OF FOOD MICROBIOLOGY, ELSEVIER BV, NL, vol. 234, 23 June 2016 (2016-06-23), pages 9-14, XP029682936, ISSN: 0168-1605, DOI: 10.1016/J.IJFOODMICRO.2016.06.024
- ROSSANA CODA ET AL: "Antifungal activity of Wickerhamomyces anomalus and Lactobacillus plantarum during sourdough fermentation: identification of novel compounds and long-term effect during storage of wheat bread.", APPLIED AND ENVIRONMENTAL MICROBIOLOGY, vol. 77, no. 10, 15 May 2011 (2011-05-15), pages 3484-3492, XP55708593, US ISSN: 0099-2240, DOI: 10.1128/AEM.02669-10
- OSHIRO MUGIHITO ET AL: "Dense tracking of the dynamics of the microbial community and chemicals constituents in spontaneous wheat sourdough during two months of backslopping", JOURNAL OF BIOSCIENCE AND BIOENGINEERING, ELSEVIER, AMSTERDAM, NL, vol. 128, no. 2, 1 August 2019 (2019-08-01), pages 170-176, XP085728523, ISSN: 1389-1723, DOI: 10.1016/J.JBIOSC.2019.02.006 [retrieved on 2019-03-14]
- LI ZHIJIAN ET AL: "Performance of non-Saccharomycesyeasts isolated from Jiaozi in dough fermentation and steamed bread making", LWT- FOOD SCIENCE AND TECHNOLOGY, vol. 111, 6 May 2019 (2019-05-06), pages 46-54, XP085714315, ISSN: 0023-6438, DOI: 10.1016/J.LWT.2019.05.019
- LIU TONGJIE ET AL: "Predominant yeasts in Chinese traditional sourdough and their influence on aroma formation in Chinese steamed bread", FOOD CHEMISTRY, ELSEVIER LTD, NL, vol. 242, 18 September 2017 (2017-09-18), pages 404-411, XP085213119, ISSN: 0308-8146, DOI: 10.1016/J.FOODCHEM.2017.09.081
- GRAEME M WALKER: "Pichia anomala: cell physiology and biotechnology relative to other yeasts", ANTONIE VAN LEEUWENHOEK, KLUWER ACADEMIC PUBLISHERS, DO, vol. 99, no. 1, 14 August 2010 (2010-08-14), pages 25-34, XP019876131, ISSN: 1572-9699, DOI: 10.1007/S10482-010-9491-8

## Description

### Technical Field

The present invention relates to the technical field of food processing, specifically to a multi-strain composite starter for flour products and use thereof.

### Background Art

With the rapid development and use of commercial dry yeast, the production of fermented flour products such as steamed bun and bread has become convenient and fast, and has also brought great convenience to people's lives. Therefore, commercial dry yeast has gradually replaced the traditional starter such as sourdough in the production process of fermented flour products, but it is undeniable that pure yeast fermentation has the disadvantage of relatively single flavor and taste of flour products. With the economic development and social progress, people have higher and higher requirements on the flavor and taste of flour products such as steamed bun and bread. The traditional sourdough fermented flour products have been welcomed by consumers with their unique flavor and taste.

The sourdough microbial flora consists of yeast, lactic acid bacteria, acetic acid bacteria, mold and other microbial flora. The metabolic process of yeast produces carbon dioxide, ethanol and small-molecule flavor substances, which endow steamed buns with unique texture and flavor characteristics. Organic acids (such as lactic acid and acetic acid) produced by metabolism of lactic acid bacteria and acetic acid bacteria and the like in dough and alcohols (such as ethanol) produced by yeast are further subjected to acetification reaction to generate a certain amount of ester compounds, and the long-term interaction of various microbial floras also produce a small amount of carbonyl compounds such as aldehydes and ketones, which constitute the important flavor volatilization and flavor auxiliary substances in sourdough. The extracellular polysaccharides and organic acids secreted by the lactic acid bacteria during the fermentation process can improve the texture of the dough, inhibit the growth of harmful microorganisms, and prolong the shelf life of the fermented products.

However, the dough fermented using the sourdough has a unique sour taste after a long period of time (6 to 12 hours) of fermentation, so the dough needs to be neutralized with alkali, which will cause the nutrients to be destroyed to some extent. At the same time, the traditional sourdough has a long production cycle, and contains infectious microbes and even cancerogenic substances such as aflatoxin, and it is difficult to be used in industrial production. In addition, the traditional sourdough is generally in a semi-solid state, is highly sensitive to microbial contamination and is not easy to be stored for a long time. Therefore, it has become a research hotspot in recent years to separate dominant strains from traditional sourdough and to compound them with yeast or other strains.

Zheng et al (2019) Food Chemistry 274:710-717 describe several natural Chinese sourdoughs comprising in combination various lactic acid bacteria, including *L. plantarum, W. anomalus* and S. *cerevisiae* in various ratio's.

### Summary of the Invention

The purpose of the present invention is to overcome the defects in the prior art and to provide a multi-strain composite starter for flour products and use thereof.

Specifically, the multi-strain composite starter for flour products according to the present invention comprises *Lactobacillus plantarum, Lactobacillus bulgaricus, Streptococcus thermophilus, Saccharomyces cerevisiae* powder, and *Wickerhamomyces anomalus* powder;
wherein, the weight ratio of *Lactobacillus plantarum, Lactobacillus bulgaricus, Streptococcus thermophilus, Saccharomyces cerevisiae* powder, and *Wickerhamomyces anomalus* powder (*Lactobacillus plantarum : Lactobacillus bulgaricus : Streptococcus thermophilus : Saccharomyces cerevisiae* powder : *Wickerhamomyces anomalus* powder) is (1-3):(0.1-3):(0.1-3):(3-9):(0.1-6).

The multi-strain composite starter for flour products according to the present invention is prepared from *Lactobacillus plantarum, Lactobacillus bulgaricus, Streptococcus thermophilus, Saccharomyces cerevisiae* powder, and *Wickerhamomyces anomalus* powder by mixing according to a specific weight ratio. The multi-strain composite starter not only solves the problems of single taste and flavor of fermented flour products (steamed bun, bread and the like) produced by using commercial yeast alone, but also solves the problems of long fermentation time, destruction of nutrients and easy generation of harmful microorganisms existing in the production of fermented flour products by using traditional sourdough.

In order to further improve the taste and flavor of the fermented flour products, shorten the fermentation time, increase the nutritional value, and prolong the shelf life, the present invention optimizes the weight ratio of the multi-strain composite starter for flour products.

Preferably, the weight ratio of *Lactobacillus plantarum, Lactobacillus bulgaricus, Streptococcus thermophilus, Saccharomyces cerevisiae* powder, and *Wickerhamomyces anomalus* powder *(Lactobacillus plantarum : Lactobacillus bulgaricus : Streptococcus thermophilus : Saccharomyces cerevisiae* powder : *Wickerhamomyces anomalus* powder) is (1-3):(0.1-1):(0.1-1):(6-9):(0.1-3).

As a preferred technical solution of the present invention, in the multi-strain composite starter for flour products according to the present invention, the weight ratio of *Lactobacillus plantarum, Lactobacillus bulgaricus, Streptococcus thermophiles, Saccharomyces cerevisiae* powder and *Wickerhamomyces anomalus* powder *(Lactobacillus plantarum : Lactobacillus bulgaricus : Streptococcus thermophilus : Saccharomyces cerevisiae* powder : *Wickerhamomyces anomalus* powder) is 2:0.5:0.5:8:1.

Alternatively, the weight ratio of *Lactobacillus plantarum, Lactobacillus bulgaricus, Streptococcus thermophilus, Saccharomyces cerevisiae* powder, and *Wickerhamomyces anomalus* powder *(Lactobacillus plantarum : Lactobacillus bulgaricus : Streptococcus thermophilus : Saccharomyces cerevisiae* powder : *Wickerhamomyces anomalus* powder) is 1:1:1:6:3.

Preferably, the *Lactobacillus plantarum* is a lyophilized *Lactobacillus plantarum;*
and/or, the *Lactobacillus bulgaricus* is a lyophilized *Lactobacillus bulgaricus;*
and/or, the *Streptococcus thermophilus* is a lyophilized *Streptococcus thermophilus.*

Preferably, the number of viable bacteria of the *Lactobacillus plantarum* is not less than 1×10¹⁰ cfu/g; and/or
the number of viable bacteria of the *Lactobacillus bulgaricus* is not less than 1×10¹⁰ cfu/g; and/or,
the number of viable bacteria of the *Streptococcus thermophilus* is not less than 1×10¹⁰ cfu/g; and/or,
the number of viable bacteria of the *Saccharomyces cerevisiae* powder is not less than 2×10¹⁰ cfu/g; and/or,
the number of viable bacteria of the *Wickerhamomyces anomalus* powder is not less than 1×10¹⁰ cfu/g.

Further, the lyophilized *Lactobacillus plantarum,* the lyophilized *Lactobacillus bulgaricus,* the lyophilized *Streptococcus thermophilus,* the *Saccharomyces cerevisiae* powder, and the *Wickerhamomyces anomalus* powder are all derived from the commercially available single viable bacteria preparation of each strain, or a bacterial preparation prepared by conventional culturing and propagation using any known strain which has been microbiologically identified as the corresponding strain.

The present invention also provides the use of the multi-strain composite starter for flour products in the production of flour products, preferably in the production of steamed bun or bread.

The present invention also provides a flour product which is produced by using any of the above multi-strain composite starter for flour products, preferably, the flour product is steamed bun or bread.

Preferably, the multi-strain composite starter for flour products accounts for 0.3% to 0.8% of the raw material powder of the flour product.

Preferably, the raw material powder is one or more selected from wheat flour, rice flour, sweet potato flour, potato flour, and coarse cereal flour.

More preferably, the raw material powder is one selected from wheat flour, wheat-sweet potato mixed flour, wheat-potato mixed flour, and wheat-sweet potato-potato mixed flour.

Preferably, the preparation method of the flour product comprises the following steps:
(1) adding the multi-strain composite starter for flour products into water and stirring well;
(2) pouring the raw material powder of the flour product into a dough mixer, and adding the mixed solution of the multi-strain composite starter for flour products obtained in the step (1); and
(3) subjecting the dough obtained in the step (2) to fermentation.

Preferably, the fermentation temperature is 30 to 35°C and the fermentation time is 30 to 60 minutes.

The beneficial effects of the present invention are as follows:
(1) The multi-strain composite starter for flour products provided by the present invention has the advantages of short fermentation time (consistent with fermentation time consumed when using commercial yeast), wide application range (applicable to the fermented flour products with wheat flour, rice flour, sweet potato flour, potato flour and coarse cereal flour as raw materials), and convenient storage and transportation.
(2) The fermented flour products such as steamed bun and bread produced using the multi-strain composite starter for flour products provided by the present invention have better taste and flavor than the flour products fermented using commercial yeast alone, and amino acid composition significantly superior to that of the flour products fermented using commercial yeast alone.

### Specific Modes for Carrying Out the Embodiments

The following Examples are intended to illustrate the present invention, but are not intended to limit the scope of the present invention.

### Example 1

The present Example provides a multi-strain composite starter for flour products, which was prepared from lyophilized *Lactobacillus plantarum,* lyophilized *Lactobacillus bulgaricus,* lyophilized *Streptococcus thermophilus, Saccharomyces cerevisiae* powder, and *Wickerhamomyces anomalus* powder by mixing according to a weight ratio of 2:0.5:0.5:8:1;
wherein, the number of viable bacteria of the lyophilized *Lactobacillus plantarum* is not less than 1×10¹⁰ cfu/g, the number of viable bacteria of the lyophilized *Lactobacillus bulgaricus* is not less than 1×10¹⁰ cfu/g, the number of viable bacteria of the lyophilized *Streptococcus thermophilus* is not less than 1×10¹⁰ cfu/g, the number of viable bacteria of the *Saccharomyces cerevisiae* powder is not less than 2×10¹⁰ cfu/g, and the number of viable bacteria of the *Wickerhamomyces anomalus* powder is not less than 1×10¹⁰ cfu/g.

Each of the strains used was derived from the commercially available single viable bacteria preparation of each strain, or a bacterial preparation prepared by conventional culturing and propagation using any known strain which has been microbiologically identified as the corresponding strain.

The present Example also provides a steamed bun. The raw material powder of the steamed bun is wheat-potato mixed flour, wherein the potato powder accounts for 30% of the total mass of the wheat-potato mixed flour.

The preparation method of the steamed bun was as follows:
(1) The multi-strain composite starter for flour products accounting for 0.8% of the weight of the wheat-potato mixed flour was accurately weighed, tap water for kneading dough was added (the amount of water added is 70% of the weight of the wheat-potato mixed flour, and the temperature of water is room temperature), and the mixture was uniformly stirred;
(2) the wheat-potato mixed flour was poured into a dough mixer, and the mixed solution of the multi-strain composite starter for flour products obtained in the step (1) was added; and
(3) the following conventional procedures were performed: kneading dough → pressing → cutting → forming → shaping → fermenting → steaming → cooling → packaging, wherein the fermentation temperature was 35°C, and the fermentation time was 40 minutes.

### Example 2

The present Example provides a multi-strain composite starter for flour products, which was the same as that of Example 1 except that, the multi-strain composite starter for flour products was prepared from lyophilized *Lactobacillus plantarum,* lyophilized *Lactobacillus bulgaricus,* lyophilized *Streptococcus thermophilus, Saccharomyces cerevisiae* powder, and *Wickerhamomyces anomalus* powder by mixing according to a weight ratio of 1:1:1:6:3.

The present Example also provides a steamed bun prepared by using the above multi-strain composite starter for flour products, and the preparation method of the steamed bun was the same as that of Example 1.

### Example 3

The present Example provides a multi-strain composite starter for flour products, and the multi-strain composite starter for flour products was the same as that of Example 1.

The present Example also provides a steamed bun prepared by using the above multi-strain composite starter for flour products, and the steamed bun was the same as that of Example 1 except that the raw material powder of the steamed bun is wheat flour.

### Comparative Example 1

The present Comparative Example provides a multi-strain composite starter for flour products, which was the same as that of Example 1 except that, the multi-strain composite starter of flour products was prepared from lyophilized *Lactobacillus plantarum,* lyophilized *Lactobacillus bulgaricus,* and *Saccharomyces cerevisiae* powder by mixing according to a weight ratio of 2:1:9.

The present Comparative Example also provides a steamed bun prepared by using the above multi-strain composite starter for flour products, and the preparation method of the steamed bun was the same as that of Example 1.

### Comparative Example 2

The present Comparative Example provides a double-strain composite starter for flour products, which was the same as that of Example 1 except that, the double-strain composite starter for flour products was prepared from lyophilized *Lactobacillus plantarum* and *Saccharomyces cerevisiae* powder by mixing according to a weight ratio of 1:3.

The present Comparative Example also provides a steamed bun prepared by using the above double-strain composite starter for flour products, and the preparation method of the steamed bun was the same as that of Example 1.

### Comparative Example 3

The present Comparative Example provides a single-strain starter of flour products, which was the same as that of Example 1 except that, the single-strain starter for flour products was *Saccharomyces cerevisiae* powder.

The present Comparative Example also provides a steamed bun prepared by using the above single-strain starter for flour products, and the steamed bun was the same as that of Example 1 except that, the single-strain starter for flour products accounts for 0.7% of the weight of the wheat-potato mixed flour.

### Comparative Example 4

The present Comparative Example provides a single-strain starter for flour products, which was the same as that of Example 1 except that, the single-strain starter for flour products was *Saccharomyces cerevisiae* powder.

The present Comparative Example also provides a steamed bun prepared by using the above single-strain starter for flour products, and the steamed bun was the same as that of Example 1 except that, the raw material powder was wheat flour, and the single-strain starter for flour products accounts for 0.7% of the weight of wheat flour.

### Comparative Example 5

The present Comparative Example provides a multi-strain composite starter for flour products, which was the same as that of Example 1 except that, the multi-strain composite starter for flour products was prepared from lyophilized *Lactobacillus plantarum,* lyophilized *Lactobacillus bulgaricus,* lyophilized *Streptococcus thermophilus, Saccharomyces cerevisiae* powder, and *Wickerhamomyces anomalus* powder by mixing according to a weight ratio of 1:3:5:1:2.

The present Comparative Example also provides a steamed bun prepared by using the above multi-strain composite starter for flour products, and the preparation method of the steamed bun was the same as that of Example 1.

### Comparative Example 6

The present Comparative Example provides a multi-strain composite starter for flour products, which was the same as that of Example 1 except that, the lyophilized *Lactobacillus plantarum* is replaced with *Lactobacillus acidophilus;* and the *Saccharomyces cerevisiae* powder is replaced with *Pichia pastoris.*

The present Comparative Example also provides a steamed bun prepared by using the above multi-strain composite starter for flour products, and the preparation method of the steamed bun was the same as that of Example 1.

### Test Example 1

In the present Test Example, the sensory and nutritional characteristics of the steamed bun samples obtained in Examples 1 to 3 and Comparative Examples 1 to 6 were analyzed by the following methods:

### (1) Analysis of apparent characteristics

The specific volume was determined by the rapeseed replacement method;

The height/diameter ratio was measured using a vernier caliper;

Texture characteristics of steamed bun slices were measured using a texture analyzer: 4 slices of each sample (taken from the center of the steamed bun, 20 mm thick) were evaluated using a cylindrical probe with a diameter of 50 mm; the pre-test speed was 2.0 mm/s, the test speed was 1.0 mm/s, the post-test speed was 2.0 mm/s, the distance was 50%, and the gap between tests was 5 seconds; and the hardness, elasticity, chewiness and cohesiveness were calculated from the obtained texture curve.

The sensory evaluation of the steamed bun was carried out in the form of questionnaire by a sensory evaluation team consisting of 14 semi-trained members (9 females and 5 males aged between 23 and 42 years) from the Institute of Food Science and Technology, Chinese Academy of Agricultural Sciences. The sensory evaluation provides a centesimal system including appearance (15%), internal structure (10%), elasticity (10%), chewiness (10%), adhesion (10%), aroma (15%), color (10%) and overall acceptability (20%). The steamed bun samples were dispensed and packed in ziplock bags by random coding, and drinking water was provided to clean the mouth during the evaluation process.

### (2) Analysis of amino acid composition

The amino acid composition of the steamed bun samples was determined by a fully automatic amino acid analyzer, and the specific steps were as follows:
10 mL of 6 N HCl was mixed with 80 mg of the sample in a Pyrex tube, followed by nitrogen purging for 1 minute; then the tube was sealed and heated at 110°C for 24 hours; the hydrolyzate was filtered through a 0.2 µm filter membrane and dried under vacuum at 60°C; the dried hydrolyzate was then dissolved in 1 mL of 0.02 N HCl and 20 µL of the resulting mixed solution was taken for analysis.

Protein quality was evaluated by chemical scoring and calculated by comparing the content of essential amino acids in the steamed bun with the values recommended in FAO 2007. The recommended levels for each essential amino acid (EAA) are as follows (g/100 g): lysine, 4.5; histidine, 1.5; threonine, 2.3; valine, 3.9; isoleucine, 3.0; leucine, 5.9; methionine, 1.6; and phenylalanine and tyrosine, 3.0.

The test data was as follows:
Table 1 shows the specific volume and height/diameter ratio of different steamed bun samples.

**Table 1: Specific volume and height/diameter ratio of different steamed bun samples**

| Steamed Bun Samples | Specific Volume (mL/g) | Height/Diameter Ratio |
|---|---|---|
| Example 1 | 2.39±0.06 | 0.72±0.01 |
| Example 2 | 2.22±0.12 | 0.72±0.04 |
| Example 3 | 2.41±0.18 | 0.70±0.00 |
| Comparative Example 1 | 2.10±0.10 | 0.69±0.02 |
| Comparative Example 2 | 1.98±0.01 | 0.69±0.03 |
| Comparative Example 3 | 1.77±0.17 | 0.66±0.01 |
| Comparative Example 4 | 2.21±0.05 | 0.71±0.03 |
| Comparative Example 5 | 1.35±0.07 | 0.65±0.04 |
| Comparative Example 6 | 1.19±0.13 | 0.65±0.02 |

As shown in Table 1, the specific volume of the steamed bun samples of Examples 1 to 3 is larger than that of the steamed bun samples of Comparative Examples 1 to 6.

Table 2 shows the texture characteristics of different steamed bun samples.

**Table 2: Texture characteristics of different steamed bun samples**

| Steamed Bun Samples | Hardness (g) | Chewiness (g) | Resilience (%) | Cohesiveness |
|---|---|---|---|---|
| Example 1 | 3380.82±163.93 | 2513.86±195.78 | 0.43±0.02 | 0.78±0.05 |
| Example 2 | 3610.53±296.43 | 2806.75±109.44 | 0.35±0.01 | 0.73±0.03 |
| Example 3 | 3409.19±71.78 | 2544.17±339.15 | 0.42±0.04 | 0.80±0.04 |
| Comparative Example 1 | 4416.83±161.65 | 2949.18±138.11 | 0.35±0.02 | 0.73±0.02 |
| Comparative Example 2 | 4420.02±99.47 | 3363.39±62.24 | 0.32±0.02 | 0.71±0.02 |
| Comparative Example 3 | 5027.92±196.80 | 3788.23±147.34 | 0.30±0.03 | 0.71±0.01 |
| Comparative Example 4 | 4115.76±101.62 | 2909.14±145.12 | 0.39±0.03 | 0.78±0.02 |
| Comparative Example 5 | 6125.45±156.73 | 3564.45±142.25 | 0.21±0.01 | 0.65±0.02 |
| Comparative Example 6 | 6587.12±123.23 | 3467.56±136.47 | 0.18±0.02 | 0.62±0.01 |

As shown in Table 2, the hardness, chewiness, and resilience of the steamed bun samples of Examples 1 to 3 are superior to those of Comparative Examples 1 to 6.

Table 3 shows the sensory scores of different steamed bun samples.

**Table 3: Sensory scores of different steamed bun samples**

| Steamed Bun Samples | Appearance | Internal Structure | Elasticity | Chewiness | Adhesion | Aroma | Color | Overall Acceptability |
|---|---|---|---|---|---|---|---|---|
| Example 1 | 14 | 9 | 8 | 9 | 8 | 15 | 10 | 19 |
| Example 2 | 14 | 8 | 8 | 8 | 8 | 13 | 9 | 18 |
| Example 3 | 15 | 9 | 10 | 9 | 9 | 12 | 10 | 17 |
| Comparative Example 1 | 13 | 8 | 7 | 8 | 7 | 13 | 9 | 18 |
| Comparative Example 2 | 11 | 8 | 6 | 7 | 7 | 11 | 9 | 16 |
| Comparative Example 3 | 10 | 6 | 5 | 5 | 7 | 10 | 7 | 13 |
| Comparative Example 4 | 14 | 8 | 9 | 9 | 9 | 11 | 8 | 15 |
| Comparative Example 5 | 8 | 5 | 4 | 4 | 5 | 8 | 7 | 9 |
| Comparative Example 6 | 8 | 4 | 4 | 3 | 3 | 5 | 5 | 7 |

As shown in Table 3, the sensory scores of the steamed bun samples of Examples 1 to 3 are superior to those of Comparative Examples 1 to 6.

Table 4 shows the amino acid composition of different steamed bun samples (g/100 g protein).

**Table 4: Amino acid composition of different steamed bun samples (g/100 g protein)**

| Total Amino Acids | Example 1 | Example 2 | Example 3 | Comparative Example 1 | Comparative Example 2 | Comparative Example 3 | Comparative Example 4 | Comparative Example 5 | Comparative Example 6 |
|---|---|---|---|---|---|---|---|---|---|
| Non-essential | | | | | | | | | |
| Amino Acids | | | | | | | | | |
| Aspartic Acid | 0.9 | 0.9 | 0.9 | 0.8 | 0.9 | 0.8 | 0.7 | 0.7 | 0.7 |
| Glutamic Acid | 3.6 | 3.7 | 3.7 | 3.7 | 3.6 | 3.7 | 3.5 | 3.4 | 3.4 |
| Serine | 0.6 | 0.6 | 0.6 | 0.6 | 0.6 | 0.6 | 0.5 | 0.5 | 0.5 |
| Glycine | 0.4 | 0.4 | 0.5 | 0.4 | 0.4 | 0.4 | 0.4 | 0.4 | 0.4 |
| Arginine | 0.5 | 0.5 | 0.6 | 0.5 | 0.5 | 0.5 | 0.5 | 0.5 | 0.5 |
| Alanine | 0.5 | 0.5 | 0.5 | 0.5 | 0.4 | 0.4 | 0.4 | 0.4 | 0.4 |
| Essential | | | | | | | | | |
| Amino Acids | | | | | | | | | |
| Threonine | 0.4 | 0.4 | 0.5 | 0.4 | 0.5 | 0.4 | 0.4 | 0.4 | 0.3 |
| Cysteine | 0.3 | 0.3 | 0.3 | 0.3 | 0.3 | 0.3 | 0.3 | 0.2 | 0.3 |
| Valine | 0.6 | 0.6 | 0.5 | 0.6 | 0.5 | 0.5 | 0.5 | 0.5 | 0.5 |
| Methionine | 0.2 | 0.2 | 0.2 | 0.2 | 0.1 | 0.2 | 0.2 | 0.1 | 0.1 |
| Isoleucine | 0.8 | 0.7 | 0.6 | 0.5 | 0.5 | 0.5 | 0.5 | 0.4 | 0.4 |
| Leucine | 1.2 | 1.1 | 1 | 1.1 | 1.1 | 1.0 | 0.9 | 0.7 | 0.8 |
| Tyrosine | 0.6 | 0.5 | 0.4 | 0.4 | 0.4 | 0.3 | 0.3 | 0.4 | 0.3 |
| Phenylalanine | 0.9 | 0.8 | 0.9 | 0.8 | 0.9 | 0.8 | 0.8 | 0.8 | 0.7 |
| Lysine | 0.5 | 0.5 | 0.6 | 0.4 | 0.4 | 0.4 | 0.4 | 0.4 | 0.4 |
| Histidine | 0.4 | 0.4 | 0.4 | 0.4 | 0.3 | 0.3 | 0.3 | 0.3 | 0.3 |
| Proline | 1.0 | 1.0 | 1.2 | 1.1 | 1.1 | 1.1 | 1.2 | 1.0 | 1.0 |
| Total Amino Acids Essential Amino | 13.3 | 13.0 | 13.4 | 12.6 | 12.7 | 12.1 | 11.7 | 11.1 | 11.0 |
| Acids/Total Amino Acids (%) | 51.1 | 49.5 | 49.3 | 48.4 | 48.5 | 47.7 | 48.8 | 46.8 | 46.4 |

As shown in Table 4, the amino acid contents of the steamed bun samples of Examples 1 to 3 are higher and the composition of the amino acids is more reasonable.

Although the present invention has been described in detail above with the aid of the general description, the specific modes for carrying out the embodiments and the tests, it may be obvious to a person skilled in the art that some modifications or improvements can be made on the basis of the present invention.

## Claims

1. A multi-strain composite starter for flour products, comprising: *Lactobacillus plantarum, Lactobacillus bulgaricus, Streptococcus thermophilus, Saccharomyces cerevisiae* powder, and *Wickerhamomyces anomalus* powder;
wherein, the weight ratio of *Lactobacillus plantarum, Lactobacillus bulgaricus, Streptococcus thermophilus, Saccharomyces cerevisiae* powder, and *Wickerhamomyces anomalus* powder is (1-3):(0.1-3):(0.1-3):(3-9):(0.1-6).

2. The multi-strain composite starter for flour products according to claim 1, wherein, the weight ratio of *Lactobacillus plantarum, Lactobacillus bulgaricus, Streptococcus thermophilus, Saccharomyces cerevisiae* powder, and *Wickerhamomyces anomalus* powder is (1-3):(0.1-1):(0.1-1):(6-9):(0.1-3).

3. The multi-strain composite starter for flour products according to claim 1 or 2, wherein, the weight ratio of *Lactobacillus plantarum, Lactobacillus bulgaricus, Streptococcus thermophilus, Saccharomyces cerevisiae* powder, and *Wickerhamomyces anomalus* powder is 2:0.5:0.5:8:1; or
the weight ratio of *Lactobacillus plantarum, Lactobacillus bulgaricus, Streptococcus thermophilus, Saccharomyces cerevisiae* powder, and *Wickerhamomyces anomalus* powder is 1:1:1:6:3.

4. The multi-strain composite starter for flour products according to any one of claims 1 to 3, wherein, the *Lactobacillus plantarum* is a lyophilized *Lactobacillus plantarum;* and/or
the *Lactobacillus bulgaricus* is a lyophilized *Lactobacillus bulgaricus*; and/or
the *Streptococcus thermophilus* is a lyophilized *Streptococcus thermophilus.*

5. The multi-strain composite starter for flour products according to any one of claims 1 to 4, wherein, the number of viable bacteria of the *Lactobacillus plantarum* is not less than 1×10¹⁰ cfu/g; and/or
the number of viable bacteria of the *Lactobacillus bulgaricus* is not less than 1×10¹⁰ cfu/g; and/or
the number of viable bacteria of the *Streptococcus thermophilus* is not less than 1×10¹⁰ cfu/g; and/or
the number of viable bacteria of the *Saccharomyces cerevisiae* powder is not less than 2×10¹⁰ cfu/g; and/or
the number of viable bacteria of the *Wickerhamomyces anomalus* powder is not less than 1×10¹⁰ cfu/g.

6. The use of the multi-strain composite starter for flour products according to any one of claims 1 to 5 in the production of flour products, preferably in the production of steamed bun or bread.

7. A flour product, wherein, the flour product is produced by using the multi-strain composite starter for flour products according to any one of claims 1 to 5, preferably, the flour product is steamed bun or bread.

8. The flour product according to claim 7, wherein, the multi-strain composite starter for flour products accounts for 0.3% to 0.8% of the raw material powder of the flour product;
preferably, the raw material powder is one or more selected from wheat flour, rice flour, sweet potato flour, potato flour, and coarse cereal flour;
more preferably, the raw material powder is one selected from wheat flour, wheat-sweet potato mixed flour, wheat-potato mixed flour, and wheat-sweet potato-potato mixed flour.

9. The flour product according to claim 7 or 8, wherein, the flour product is prepared by a method comprising the following steps:
(1) adding the multi-strain composite starter for flour products into water and stirring well;
(2) pouring the raw material powder of the flour product into a dough mixer, and adding the mixed solution of the multi-strain composite starter for flour products obtained in the step (1); and
(3) subjecting the dough obtained in the step (2) to fermentation.

10. The flour product according to claim 9, wherein, the fermentation temperature is 30 to 35°C and the fermentation time is 30 to 60 minutes.

## Patentansprüche

1. Multistamm-Mischungsstarter für Mehlprodukte umfassend: *Lactobacillus plantarum, Lactobacillus bulgaricus, Streptococcus thermophilus, Saccharomyces cerevisiae-Pulver* und *Wickerhamomyces anomalus*-Pulver;
wobei das Gewichtsverhältnis von *Lactobacillus plantarum, Lactobacillus bulgaricus, Streptococcus thermophilus, Saccharomyces cerevisiae-Pulver* und *Wickerhamomyces anomalus-*Pulver (1-3):(0,1-3):(0,1-3):(3-9):(0,1-6) beträgt.

2. Der Multistamm-Mischungsstarter für Mehlprodukte gemäß Anspruch 1, wobei das Gewichtsverhältnis von *Lactobacillus plantarum, Lactobacillus bulgaricus, Streptococcus thermophilus, Saccharomyces cerevisiae-Pulver* und *Wickerhamomyces anomalus*-Pulver (1-3):(0,1-1):(0,1-1):(6-9):(0,1-3) beträgt.

3. Der Multistamm-Mischungsstarter für Mehlprodukte gemäß Anspruch 1 oder 2, wobei das Gewichtsverhältnis von *Lactobacillus plantarum, Lactobacillus bulgaricus, Streptococcus thermophilus, Saccharomyces cerevisiae-Pulver* und *Wickerhamomyces anomalus*-Pulver 2:0,5:0,5:8:1 beträgt; oder
das Gewichtsverhältnis von *Lactobacillus plantarum, Lactobacillus bulgaricus, Streptococcus thermophilus, Saccharomyces cerevisiae-Pulver* und *Wickerhamomyces anomalus*-Pulver 1:1:1:6:3 beträgt.

4. Der Multistamm-Mischungsstarter für Mehlprodukte gemäß einem der Ansprüche 1 bis 3, wobei der *Lactobacillus plantarum* ein lyophilisierter *Lactobacillus plantarum* ist; und/oder
der *Lactobacillus bulgaricus* ein lyophilisierter *Lactobacillus bulgaricus* ist; und/oder
der *Streptococcus thermophilus* ein lyophilisierter *Streptococcus thermophilus* ist.

5. Der Multistamm-Mischungsstarter für Mehlprodukte gemäß einem der Ansprüche 1 bis 4, wobei die Anzahl der lebensfähigen Bakterien des *Lactobacillus plantarum* nicht weniger als 1×10¹⁰ KbE/g beträgt; und/oder
die Anzahl der lebensfähigen Bakterien des *Lactobacillus bulgaricus* nicht weniger als 1×10¹⁰ KbE/g beträgt; und/oder
die Anzahl der lebensfähigen Bakterien des *Streptococcus thermophilus* nicht weniger als 1×10¹⁰ KbE/g beträgt; und/oder
die Anzahl der lebensfähigen Bakterien des *Saccharomyces cerevisiae-Pulvers* nicht weniger als 2×10¹⁰ KbE/g beträgt; und/oder
die Anzahl der lebensfähigen Bakterien des *Wickerhamomyces anomalus*-Pulvers nicht weniger als 1×10¹⁰ KbE/g beträgt.

6. Verwendung des Multistamm-Mischungsstarters für Mehlprodukte gemäß einem der Ansprüche 1 bis 5 bei der Herstellung von Mehlprodukten, bevorzugt bei der Herstellung von Dampfbrötchen oder Dampfbrot.

7. Ein Mehlprodukt, wobei das Mehlprodukt durch Verwendung des Multistamm-Mischungsstarters für Mehlprodukte gemäß einem der Ansprüche 1 bis 5 hergestellt wird, wobei das Mehlprodukt bevorzugt Dampfbrötchen oder Dampfbrot ist.

8. Das Mehlprodukt gemäß Anspruch 7, wobei der Multistamm-Mischungsstarter für Mehlprodukte 0,3% bis 0,8% des Ausgangsmaterials des Mehlprodukts ausmacht;
bevorzugt ist das Ausgangsmaterial eines oder mehrere der folgenden Produkte: Weizenmehl, Reismehl, Süßkartoffelmehl, Kartoffelmehl und grobes Getreidemehl;
besonders bevorzugt wird das Ausgangsmaterial ausgewählt aus Weizenmehl, Weizen-Süßkartoffel-Mischmehl, Weizen-Kartoffel-Mischmehl und Weizen-Süßkartoffel-Kartoffel-Mischmehl.

9. Das Mehlprodukt gemäß Anspruch 7 oder 8, wobei das Mehlprodukt nach einem Verfahren hergestellt wird, das die folgenden Schritte umfasst:
(1) Zugabe des Multistamm-Mischungsstarters für Mehlprodukte in Wasser und vollständiges Umrühren;
(2) Gießen des Ausgangsmaterials des Mehlprodukts in einen Teigmischer und Zugabe der gemischten Lösung des Multistamm-Mischungsstarters für Mehlprodukte, die in Schritt (1) erhalten wurde; und
(3) der im Schritt (2) erhaltene Teig wird einer Gärung unterzogen.

10. Das Mehlprodukt gemäß Anspruch 9, wobei die Fermentationstemperatur 30 bis 35°C und die Gärungszeit 30 bis 60 Minuten beträgt.

## Revendications

1. Starter composite multisouche pour des produits de farine, comprenant : *Lactobacillus plantarum, Lactobacillus bulgaricus, Streptococcus thermophilus,* une poudre de *Saccharomyces cerevisiae,* et une poudre de *Wickerhamomyces anomalus* ;
le rapport en poids de *Lactobacillus plantarum, Lactobacillus bulgaricus, Streptococcus thermophilus,* poudre de *Saccharomyces cerevisiae,* et poudre de *Wickerhamomyces anomalus* étant de (1 à 3) : (0,1 à 3) : (0,1 à 3) : (3 à 9) : (0,1 à 6).

2. Starter composite multisouche pour des produits de farine selon la revendication 1, le rapport en poids de *Lactobacillus plantarum, Lactobacillus bulgaricus, Streptococcus thermophilus,* poudre de *Saccharomyces cerevisiae,* et poudre de *Wickerhamomyces anomalus* étant de (1 à 3) : (0,1 à 1) : (0,1 à 1) : (6 à 9) : (0,1 à 3).

3. Starter composite multisouche pour des produits de farine selon la revendication 1 ou 2, le rapport en poids de *Lactobacillus plantarum, Lactobacillus bulgaricus, Streptococcus thermophilus,* poudre de *Saccharomyces cerevisiae,* et poudre de *Wickerhamomyces anomalus* étant de 2 : 0,5 : 0,5 : 8 : 1 ; ou
le rapport en poids de *Lactobacillus plantarum, Lactobacillus bulgaricus, Streptococcus thermophilus,* poudre de *Saccharomyces cerevisiae,* et poudre de *Wickerhamomyces anomalus* étant de 1 : 1 : 1 : 6 : 3.

4. Starter composite multisouche pour des produits de farine selon l'une quelconque des revendications 1 à 3, le *Lactobacillus plantarum* étant un *Lactobacillus plantarum* lyophilisé ; et/ou
le *Lactobacillus bulgaricus* étant un *Lactobacillus bulgaricus* lyophilisé ; et/ou
le *Streptococcus thermophilus* étant un *Streptococcus thermophilus* lyophilisé.

5. Starter composite multisouche pour des produits de farine selon l'une quelconque des revendications 1 à 4, le nombre de bactéries viables du *Lactobacillus plantarum* n'étant pas inférieur à 1 × 10¹⁰ ufc/g ; et/ou
le nombre de bactéries viables du *Lactobacillus bulgaricus* n'étant pas inférieur à 1 × 10¹⁰ ufc/g ; et/ou
le nombre de bactéries viables du *Streptococcus thermophilus* n'étant pas inférieur à 1 × 10¹⁰ ufc/g ; et/ou
le nombre de bactéries viables de la poudre de *Saccharomyces cerevisiae* n'étant pas inférieur à 2 × 10¹⁰ ufc/g ; et/ou
le nombre de bactéries viables de la poudre de *Wickerhamomyces anomalus* n'étant pas inférieur à 1 × 10¹⁰ ufc/g.

6. Utilisation du starter composite multisouche pour des produits de farine selon l'une quelconque des revendications 1 à 5 dans la production de produits de farine, préférablement dans la production de brioche ou pain à la vapeur.

7. Produit de farine, le produit de farine étant produit en utilisant le starter composite multisouche pour des produits de farine selon l'une quelconque des revendications 1 à 5, préférablement le produit de farine étant de la brioche ou du pain à la vapeur.

8. Produit de farine selon la revendication 7, le starter composite multisouche pour des produits de farine représentant 0,3 % à 0,8 % de la poudre de matière première du produit de farine ;
préférablement, la poudre de matière première étant l'une ou plusieurs choisie(s) parmi de la farine de blé, de la farine de riz, de la farine de patate douce, de la farine de pomme de terre, et de la farine de céréales grossières ;
plus préférablement, la poudre de matière première étant l'une choisie parmi la farine de blé, la farine mixte de blé-patate douce, la farine mixte de blé-pomme de terre, et la farine mixte de blé-patate douce-pomme de terre.

9. Produit de farine selon la revendication 7 ou 8, le produit de farine étant préparé par un procédé comprenant les étapes suivantes :
(1) ajout du starter composite multisouche pour des produits de farine dans de l'eau et bonne agitation ;
(2) versement de la poudre de matière première du produit de farine dans un mélangeur à pâte, et ajout de la solution mélangée du starter composite multisouche pour des produits de farine obtenu dans l'étape (1) ; et
(3) soumission de la pâte obtenue dans l'étape (2) à une fermentation.

10. Produit de farine selon la revendication 9, la température de fermentation étant de 30 à 35 °C et le temps de fermentation étant de 30 à 60 minutes.
